# EUROPEAN PATENT SPECIFICATION

(11) **EP 1 964 547 B1**
(45) Date of publication and mention of the grant of the patent: **30.09.2020**
(21) Application number: 07120112.3
(22) Date of filing: 06.11.2007
(51) Int. Cl.: A61K 9/00, A61K 47/32

(54) **Adhesive topical composition having a barrier action on aphthous ulcers of the oral mucosa**
Haftende topische Zusammensetzung mit einer Barrierewirkung auf Aphthen der Mundschleimhaut
Composition topique adhésive ayant un effet barrière sur les ulcèrations aphteuses de la muqueuse buccale

(30) Priority: 20.11.2006 IT RM20060624
(43) Date of publication of application: 03.09.2008
(73) Proprietor: D.M.G. Italia Srl, 00071 Pomezia (RM) (IT)
(72) Inventor: Mercuri, Luigi, I-00040, Pomezia (RM) (IT)
(74) Representative: Hoffmann Eitle

(56) References cited:
- EP-A- 0 406 643
- GB-A- 2 392 384
- US-A- 4 948 580
- US-A- 5 714 165
- US-B1- 6 352 711

## Description

The present invention relates to an adhesive topical composition dedicated to the oral mucosa, having a protective, lenitive, and antibacterial action on aphthous ulcers.

More in particular, the invention relates to an adhesive composition for the topical treatment of aphthous ulcers, which, thanks to its capacity to adhere to the mucosae, forms on the lesion an adherent protective film that enables protection of the lesion itself from external agents and enables the active principles used to remain longer in contact, at an optimal concentration, with the aphthous ulcer, so as to perform its own action in the best possible way.

According to the invention, said adhesive topical formulation is in the form of an anhydrous base containing polymers that, in contact with the moisture of the oral mucosa, are able to develop, in a few seconds, an evident adhesive capacity that enables the product to remain adherent to the surface in the point of application and to exert a protective action in regard to external agents. It should be emphasized that the polymers used meet all the safety requirements for being applied in the oral cavity.

Solubilized and/or dispersed within the anhydrous base are active principles chosen from among the ones that are able to protect the surface, soothe pain, and exert an antibacterial action.

Comprised among the active principles the primary action of which is to protect the surface and soothe pain are: acid 18 beta-glycyrrhetic acid, carboxymethyl betaglucan, d-panthenol, aloe and mallow, zinc oxide, and vitamins A and E.

The preservative system, which is able to ensure an antibacterial activity in the point of application, is instead constituted by xylitol, sodium benzoate, and chlorobutanol.

The composition of the present invention, for topical use in the therapeutic treatment of aphthous ulcer of the oral mucosa, is characterized in that it comprises:
an adhesive component constituted by a polymer that is able to develop an evident adhesive capacity in contact with the oral mucosa, preferentially a mixed salt of calcium/sodium of a copolymer of a methylvinylether - maleic-anhydride type;
a thickening component that comprises carboxymethylcellulose;
a component operating as substrate or vehicle that comprises a mixture of vaseline oil and white vaseline;
an antibacterial and antiseptic component comprising xylitol;
a preservative component comprising chlorobutanol and sodium benzoate;
an anti-inflammatory, emollient, and lenitive component comprising mallow extract and stearyl glycyrrhizate;
an antioxidant component comprising vitamin E acetate, vitamin A palmitate, and D-panthenol; and adjuvants comprising colorants and aromatizing agents.

The amounts of ingredients in each component of the composition that is described can be varied within certain ranges to provide adhesive products for the therapeutic treatment of aphthous ulcer suitable for the use according to the invention.

The present invention also concerns a process with which these adhesive products for the therapeutic treatment of aphthous ulcer can be prepared.

### Description of the known art

Aphthosis is one of the most common diseases of the oral cavity, and it is calculated that 30 to 40% of the population presents this disorder in the course of their life.

The precise cause of the affection is unknown: no known bacterium or virus has emerged as being responsible for the disease. It is deemed that it may depend upon a genetic predisposition since, in approximately one third of cases, family history is positive. The most frequently associated conditions are deficiency of iron, folic acid, and vitamin B12, coeliac disease, Crohn's disease, pernicious anaemia, forms of multiple immunodeficiency (e.g., HIV infection).

Initially, aphthous ulcer presents as a roundish erosion, which is very painful, shallow, with clear margins and a yellowish rim surrounded by an erythematous halo; the bottom is greyish yellow.
The smaller ulcers have a diameter of 2-4 mm, number from 1 to 6, and, after a phase of acute pain of 3-5 days, heal without cicatrizing in approximately 10 days.

The larger ulcers, having a diameter of up to 10 mm, mend more slowly, with evident cicatricial results.

The site of the lesions is above all the margin and the ventral surface of the tongue, the soft palate, the mucosa, the buccal floor, and the labial vestibule. Only rarely are the hard palate and the fixed gum involved, which are sites, instead, of recurrent intraoral herpetic ulcers.

The therapies proposed are numerous and comprise topical and systemic treatments.

Topical medicaments comprise anaesthetic, antihistaminic, antimicrobial, and anti-inflammatory agents. However, the confirmation of an effective use of these agents for aphthous ulcers is substantially anecdotal. In limited tests, beta-methasone gel, beta-clomethasone spray and amlexanox have led to significant reductions in aphthous ulcers but have not led to any modification in the frequency and seriousness of the recurrences. Basically, the topical medicaments have proven far from effective on account of the presence of salivary secretion, which exerts a flushing action and reduces the time of contact of the active principles on the lesion of the mucosa.

Systemic treatments comprise corticosteroids in oral administration, colchicine, levamisole, and thalidomide. Such treatments present, however, marked contraindications both on account of the frequent negative side effects, such as somnolence, nausea, tiredness, and on account of their toxicity.

Practical experience has moreover suggested conversion of the aphthous ulcer into a lesion by means of causticization of the aphthous ulcer obtained with chemical or physical means (laser, silver nitrate, etc.). It is evident, however, that recourse to causticization is traumatic and not always proposable.

From the foregoing, there emerges the need for a different approach in the treatment of aphthous ulcers.

Following upon the experiments conducted, there has emerged the fact that a composition for the topical treatment of aphthous ulcer of the oral mucosa containing a high percentage of a mixed partial salt of calcium/sodium of a copolymer of a methylvinylether - maleic-anhydride type, is able to exert a barrier action on the aphthous ulcers with a dual effect:
a) it guarantees that the lesion will be protected from the external agents for a suitable period of time; and
b) simultaneously, it enables the active principles contained in the composition itself to perform their action in the best possible way, keeping them in contact with the lesion at an optimal concentration.

Consequently, forming a first subject of the present invention is a composition for topical use in the therapeutic treatment of aphthous ulcer of the oral mucosa having a barrier effect comprising a mixed partial salt of calcium/sodium of a copolymer of a methylvinylether - maleic-anhydride type, as adhesivizing agent, together with antibacterial agents, decongestioning agents, and adjuvants for use in the topical treatment of aphthous ulcer of the oral mucosa.

Forming another subject of the present description is the use of a mixed partial salt of calcium/sodium of a copolymer of a methylvinylether - maleic-anhydride type for the preparation of a topical composition having a barrier effect for the topical treatment of aphthous ulcer of the oral mucosa.

A convenient preferred mixed partial salt of calcium/sodium of a copolymer of a methylvinylether - maleic-anhydride type is Gantrez MS955, a commercially available product, manufactured by ISP (International Specialty Products).

Gantrez MS955 is a biocompatible vinyl copolymer, in powder form, which is liposoluble and develops its adhesive power once it has come into contact with water.

Said substance is normally used for the preparation of adhesive paste for dentures, toothpastes and collutories in so far as it provides superior adhesive properties for prolonged periods of time as compared to other polymeric materials.

It should be recalled that the adhesive compositions for dental prostheses are generally prepared from natural or synthetic polymeric materials suspended in a system operating as oleaginous vehicle.

Following upon contact with water, these polymeric materials have the property of undergoing impregnation up to several times their own original volume to form a gelatinous mass that can fill the space, act as buffer, and provide adhesion between the plaque of a dental prosthesis and the tissue of the gum.

Likewise known is a toothpaste that contains a
combination of Gantrez with an antibacterial agent, triclosan, which is used as an aid to prevention of oral plaque and gengivitis. Gantrez enables the triclosan to remain on the surface of the teeth and the gums and perform its action for over twelve hours.

No reference exists in the literature regarding the use of Gantrez MS955 for the production of a medicament for the treatment of aphthous ulcer.

In a preferred embodiment, the composition forming the subject of the invention comprises a substantially anhydrous mixture that includes (expressed in weight percentages):
a) from 5 wt% to 45 wt% of said mixed partial salt of calcium/sodium of a copolymer of methylvinylether and maleic anhydride;
b) from 10 wt% to 40 wt% of vaseline oil;
c) from 10 wt% to 40 wt% of white vaseline;
d) from 2 wt% to 25 wt% of carboxymethylcellulose;
e) from 1 wt% to 10 wt% of xylitol;
f) from 0,5 wt% to 10 wt% of d-panthenol;
g) from 0,1 wt% to 5 wt% of carboxymethyl betaglucan;
h) from 0,1 wt% to 5 wt% of lyophilized aloe vera;
i) from 0,1 wt% to 5 wt% of liposoluble mallow extract;
j) from 0,05 wt% to 0,3 wt% of sodium benzoate;
l) from 0,01 wt% to 2 wt% of vitamin E acetate;
m) from 0,01 wt% to 3 wt% of stearyl glycyrrhizate;
n) from 0,01 wt% to 2 wt% of vitamin A palmitate;
o) from 0,1 wt% to 5 wt% of zinc oxide;
p) from 0,1 wt% to 2 wt% of chlorobutanol;
q) from 0,000001 wt% to 0,0001 wt% of green colorant; and
r) from 0,05 wt% to 1 wt% of mint aroma.

The ratio between the amounts of the ingredients contained in the adhesive component, the mixed partial salt of calcium/sodium of a copolymer of methylvinylether and maleic anhydride and of carboxymethylcellulose can be made to vary within given ranges to provide adhesive products convenient for use in the treatment of aphthous ulcer.

Preferably, the mixed partial salt of calcium/sodium of a methylvinylether and maleic-anhydride copolymer and carboxymethylcellulose are present in a weight ratio of between 2,8:1 and 1,5:1, respectively, more preferentially in a weight ratio of between 2,6:1 and 2:1.

The ratio between the amounts of the ingredients in the substrate component, vaseline, and vaseline oil can be varied within certain ranges to provide adhesive products for the treatment of aphthous ulcer convenient for the application.

Preferably, vaseline oil and vaseline are present in a weight ratio comprised between 1,5:1 and approximately 1:1, more preferentially in a weight ratio of between 1,2:1 and 1:1.

The partial mixed salts of calcium and sodium of methylvinylether - maleic-anhydride copolymers and the techniques of polymerization used to obtain these copolymers are well known in the sector. The molar ratio between the monomeric component of methylvinylether and the monomeric component of maleic anhydride is substantially unitary.

According to the invention, the problem of the poor dispersion of xylitol has been solved using the latter after being subjected to a process of micronization. In this way, it has been possible to disperse the xylitol in a more homogeneous way, without using dispersing means, such as glycerine or glycol.

There has thus been identified a qualitative formulation of an anhydrous paste for the treatment of aphthous ulcer, as follows:
xylitol
d-panthenol
carboxymethyl betaglucan
lyophilized aloe vera
liposoluble mallow extract
vitamin E acetate
stearyl glycyrrhizate
vitamin A palmitate
zinc oxide
Gantrez MS955
vaseline oil
white vaseline
carboxymethylcellulose
sodium benzoate
chlorobutanol
green colorant
mint aroma
where some of the components exert, alongside the primary action of protecting the lesioned surface and soothing the pain, also a secondary action linked to their antibacterial, immunological, and re-epithelizing activities.

Indicated briefly hereinafter are the characteristics of the active principles referred to above.

Xylitol is a polyalcohol derived from the sugar xylose. It is present in nature in some fruits and vegetables, such as plums, strawberries, raspberries, and cauliflowers. It is used as non-carcinogenic and antibacterial sweetening agent in tablets and syrups and is used for oral hygiene. It also performs the function of antibacterial preservative.
Stearyl glycyrrhizate is an ester of 18 beta-glycyrrhetic acid. The latter, also known as enoxolone, is extracted from liquorice. It is an anti-inflammatory agent for topical use in skin diseases. The antilysosome action of 18 beta-glycyrrhetic acid plays a fundamental role in this property: it is in fact similar to endogenous cortisones, and thus has a similar action, especially in regard to the glomerules of the adrenal gland. On account of its poor solubility it is used in the form of a salt or an ester, as in the present case.

Dex-panthenol is an exogenous source of pantothenic acid, a vitamin that is indispensable for the intermediate metabolism of carbohydrates, proteins, and lipids. It is employed for topical use, in order to alleviate the sensation of burning and promote healing of lesions thanks to its re-epithelizing capacity.

Glucan is a polysaccharide obtained by fermentation from cultures of Saccharomyces cerevisiae. It favours repair of damaged tissues. Glucan has a marked immunostimulating power: in fact, it activates cellular differentiation, stimulating the immune system, and favours cicatrization.

Zinc oxide has astringent, protective, and antiseptic properties. It is used topically as lenitive and protective agent in eczemas and in slight excoriations. It is also used traditionally in the treatment of skin affections, such as acne, ulcers, seborrhoea, pruritis, dryness, impetigo, psoriasis, as well as in the case of insect stings and bites, and burns.

Liposoluble vitamin A is found in nature in the form of retinol and carotenoids. At an epidermal level it favours regenerative processes of the skin, increasing the resistance thereof. Shortage of vitamin A causes keratization, drying and desquamation of the skin. Vitamin A performs numerous biological functions, amongst which cellular differentiation, aids resistance of the organism, and serves also as antioxidant.

Vitamin E, which is also liposoluble, is used topically as anti-inflammatory agent. Its primary role is to protect the tissues from harmful reactions (oxidation), but its fundamental role in the product forming the subject of the invention is to protect it and also to protect vitamin A from oxidation since vitamin E is oxidized more rapidly and more easily than vitamin A.

Aloe is a succulent plant that has numerous properties. It is in fact disintoxicating, bactericidal, immunostimulating, is an aid against free radicals, increases the regenerative processes of the cells, and slows down the processes of cellular ageing, favouring elimination of free radicals.

Mallow is a plant indicated for all persons, both adults and children: it contains mucilages that are useful against inflammatory states of the mucosae, aphthous ulcer, and as collutories. It is also used for ulcers and lesions, contains the vitamin complexes B1, B2 and C, carotene, potassium, tannins, and flavonoids, the combination of which combats viruses and bacteria. Mallow also presents lenitive, softening, emollient, and soothing properties. It is a complete and safe plant, altogether suitable as lubricant for a product aimed at the oral mucosae.

Chlorobutanol is already used in preparations for the oral mucosa, is a liposoluble preservative, and does not present any incompatibility with the other ingredients.

Sodium benzoate is a safe-to-use preservative for foodstuffs.

Carbomethylcellulose is used as excipient and gelling agent in the preparation of gels. It finds use in the protection of oral and perioral lesions, where it performs a mechanical action.

Vaseline and vaseline oil are lubricating thickening vehicles used as base for ointments and bestow a creamy consistency on the product.

The aroma is added to render the taste of the product more pleasant.

Any other convenient conventional additive that is generally employed in adhesive compositions for dental prostheses may, however, be used.

Such additives comprise aromatizing agents, colouring agents, preservatives, odorants, deodorants, natural and synthetic sweeteners, antimicrobial agents, agents used in the healing of tissues, and the like. Preferably, said additives, when present, are used in amounts of up to 2 wt% of the total composition.

By way of example, provided hereinafter is the composition of a preferred embodiment of the invention.

### ANHYDROUS PASTE FOR APHTHOUS ULCER

Quali-quantitative formula per 100 grams:
a) partially mixed salt of calcium/sodium of a copolymer of methylvinylether and maleic anhydride = 25 g;
b) vaseline oil = 28,65 g;
c) white vaseline = 24 g;
d) carboxymethylcellulose = 10 g;
e) xylitol = 2 g;
f) d-panthenol = 5 g;
g) carboxymethyl betaglucan = 0,2 g;
h) lyophilized aloe vera = 1 g;
i) liposoluble mallow extract = 2 g;
j) sodium benzoate = 0,25 g;
l) vitamin E acetate = 0,5 g;
m) stearyl glycyrrhizate = 0,3 g;
n) vitamin A palmitate = 0,1 g;
o) zinc oxide = 0,2 g;
p) chlorobutanol = 0,5 g;
q) green colorant = 0,0001 g;
r) mint aroma = 0,3 g.

The present invention extends to a process for the production of an adhesive composition for topical use in the treatment of aphthous ulcer according to claim 2. The final adhesive compositions are prepared using processes generally known in the technology of adhesives for dental prostheses. In one such procedure, an adhesive composition is produced by preparing a mixture obtained by mixing the ingredients of the adhesive component and the ingredients of the hydrating and gluing component in the ingredients of the substrate component in a mixing vessel. Optional additives, such as colouring agents, preservatives, and aromatizing agents can also be mixed together.

As regards xylitol, which performs the dual function of disinfectant and sweetening agent, it is dispersed homogeneously in the substrate ingredients after undergoing a process of micronization, according to techniques that are well known in the pharmaceutical sector. After the treatment of micronization, the larger particles are dispersed, and their dimensions are reduced to obtain a product with particles comprised between 0.4 and 1 micron according to the application and the pressure in the micronization valve.

In this way, it is possible to disperse the xylitol in a more homogeneous way, without using dispersing means such as glycerine or glycol.

## Claims

1. A composition for topical use in the therapeutic treatment of aphthous ulcer of the oral mucosa **characterized in that** it comprises:
- a substantially anhydrous mixture including a mixed partial salt of calcium/sodium of a copolymer of methylvinylether and maleic anhydride;
- carboxymethylcellulose;
- vaseline oil and white vaseline;
- xylitol;
- chlorobutanol and sodium benzoate;
- mallow extract and stearyl glycyrrhizate;
- vitamin E acetate, vitamin A palmitate, and D-panthenol;
- carboxymethylbetaglucan, lyophilized aloe vera, zinc oxide;
- adjuvants comprising colorants and aromatizing agents.

2. The composition for topical use according to claim 1, **characterized in that** it includes (expressed in weight percentages):
a) 5 to 45 wt% of said substantially anhydrous mixture including the mixed partial salt of calcium/sodium of a copolymer of methylvinylether and maleic anhydride;
b) 10 to 40 wt% of vaseline oil;
c) 10 to 40 wt% of white vaseline;
d) 2 to 25 wt% of carboxymethylcellulose;
e) 1 to 10 wt% of xylitol;
f) 0.5 to 10 wt% of d-panthenol;
g) 0.1 to 5 wt% of carboxymethyl betaglucan;
h) 0.1 to 5 wt% of lyophilized aloe vera;
i) 0.1 to 5 wt% of liposoluble mallow extract;
j) 0.05 to 0.3 wt% of sodium benzoate;
l) 0.01 to 2 wt% of vitamin E acetate;
m) 0.01 to 3 wt% of stearyl glycyrrhizate;
n) 0.01 to 2 wt% of vitamin A palmitate;
o) 0.1 to 5 wt% of zinc oxide;
p) 0.1 to 2 wt% of chlorobutanol;
q) 0.000001 to 0.0001 wt% of green colorant;
r) 0.05 to 1 wt% of mint aroma.

3. The composition for topical use according to claim 2, **characterized in that** it contains (qualitative-quantitative formula per 100 grams):
a) partially mixed salt of calcium/sodium of a copolymer of methylvinylether and maleic anhydride = 25 g;
b) vaseline oil = 28.65 g;
c) white vaseline = 24 g;
d) carboxymethylcellulose = 10 g;
e) xylitol = 2 g;
f) d-panthenol = 5 g;
g) carboxymethyl betaglucan = 0.2 g;
h) lyophilized aloe vera = 1 g;
i) liposoluble mallow extract = 2 g;
j) sodium benzoate = 0.25 g;
l) vitamin E acetate = 0.5 g;
m) stearyl glycyrrhizate = 0.3 g;
n) vitamin A palmitate = 0.1 g;
o) zinc oxide = 0.2 g;
p) chlorobutanol = 0.5 g;
q) green colorant = 0.0001 g;
r) mint aroma = 0.3 g.

4. The composition for topical use according to claim 1, **characterized in that** the mixed partial salt of calcium/sodium of a copolymer of methylvinylether and maleic anhydride and the carboxymethylcellulose are present in a weight ratio between 2.8:1 and 1.5:1.

5. The composition for topical use according to claim 1, **characterized in that** vaseline and vaseline oil are present in a weight ratio between 1.5:1 and 1:1.

6. A process for the preparation of a topical adhesive composition in the therapeutic treatment of aphthous ulcer of the oral mucosa in the form of anhydrous paste consisting of the following steps:
I) mixing the following ingredients:
a) 5 to 45 wt% of a partially mixed salt of calcium/sodium of a copolymer of methylvinylether and maleic anhydride;
b) 10 to 40 wt% of vaseline oil;
c) 10 to 40 wt% of white vaseline;
d) 2 to 25 wt% of carboxymethylcellulose;
f) 0.5 to 10 wt% of d-panthenol;
g) 0.1 to 5 wt% of carboxymethyl betaglucan;
h) 0.1 to 5 wt% of lyophilized aloe vera;
i) 0.1 to 5 wt% of liposoluble mallow extract;
j) 0.05 to 0.3 wt% of sodium benzoate;
l) 0.01 to 2 wt% of vitamin E acetate;
m) 0.01 to 3 wt% of stearyl glycyrrhizate;
n) 0.01 to 2 wt% of vitamin A palmitate;
o) 0.1 to 5 wt% of zinc oxide;
p) 0.1 to 2 wt% of chlorobutanol;
g) 0.000001 to 0.0001 wt% of green colorant;
r) 0.05 to 1 wt% of mint aroma;
II) micronizing
e) 1 to 10 wt% of xylitol;
III) adding the micronized xylitol to the ingredient mixture of step I) and mixing.

## Patentansprüche

1. Zusammensetzung zur topischen Verwendung bei der therapeutischen Behandlung von Aphthen der Mundschleimhaut, **dadurch gekennzeichnet, dass** sie Folgendes umfasst:
- eine im wesentlichen wasserfreie Mischung mit einem gemischten Calcium-/Natrium-Teilsalz eines Copolymers von Methylvinylether und Maleinsäureanhydrid;
- Carboxymethylcellulose;
- Vaselinöl und weiße Vaseline;
- Xylit;
- Chlorbutanol und Natriumbenzoat;
- Malvenextrakt und Stearyl-Glycyrrhizat;
- Vitamin-E-Acetat, Vitamin-A-Palmitat und D-Panthenol;
- Carboxymethylbetaglucan, gefriergetrocknete Aloe vera, Zinkoxid;
- Hilfsstoffe umfassend Farb- und Aromastoffe.

2. Zusammensetzung zur topischen Verwendung nach Anspruch 1, **dadurch gekennzeichnet, dass** sie Folgendes enthält (Angaben in Gewichtsprozent):
a) 5 bis 45 Gewichtsprozent der im wesentlichen wasserfreien Mischung mit einem gemischten Calcium-/Natrium-Teilsalz eines Copolymers von Methylvinylether und Maleinsäureanhydrid;
b) 10 bis 40 Gewichtsprozent Vaselinöl;
c) 10 bis 40 Gewichtsprozent weiße Vaseline;
d) 2 bis 25 Gewichtsprozent Carboxymethylcellulose;
e) 1 bis 10 Gewichtsprozent Xylit;
f) 0,5 bis 10 Gewichtsprozent D-Panthenol;
g) 0,1 bis 5 Gewichtsprozent Carboxymethylbetaglucan;
h) 0,1 bis 5 Gewichtsprozent gefriergetrocknete Aloe vera;
i) 0,1 bis 5 Gewichtsprozent fettlöslichen Malvenextrakt;
j) 0,05 bis 0,3 Gewichtsprozent Natriumbenzoat;
l) 0,01 bis 2 Gewichtsprozent Vitamin-E-Acetat;
m) 0,01 bis 3 Gewichtsprozent Stearyl-Glycyrrhizat;
n) 0,01 bis 2 Gewichtsprozent Vitamin-A-Palmitat;
o) 0,1 bis 5 Gewichtsprozent Zinkoxid;
p) 0,1 bis 2 Gewichtsprozent Chlorbutanol;
q) 0,000001 bis 0,0001 Gewichtsprozent grüner Farbstoff;
r) 0,05 bis 1 Gewichtsprozent Minzaroma.

3. Zusammensetzung zur topischen Verwendung nach Anspruch 2, **dadurch gekennzeichnet, dass** sie Folgendes enthält (qualitativ-quantitative Rezeptur je 100 Gramm):
a) gemischtes Calcium-/Natrium-Teilsalz eines Copolymers von Methylvinylether und Maleinsäureanhydrid = 25 g;
b) Vaselinöl = 28,65 g;
c) weiße Vaseline = 24 g;
d) Carboxymethylcellulose = 10 g;
e) Xylit = 2 g;
f) D-Panthenol = 5 g;
g) Carboxymethylbetaglucan = 0,2 g;
h) gefriergetrocknete Aloe vera = 1 g;
i) fettlöslicher Malvenextrakt = 2 g;
j) Natriumbenzoat = 0,25 g;
l) Vitamin-E-Acetat = 0,5 g;
m) Stearyl-Glycyrrhizat = 0,3 g;
n) Vitamin-A-Palmitat = 0,1 g;
o) Zinkoxid = 0,2 g;
p) Chlorbutanol = 0,5 g;
q) grüner Farbstoff = 0,0001 g;
r) Minzaroma = 0,3 g.

4. Zusammensetzung zur topischen Verwendung nach Anspruch 1, **dadurch gekennzeichnet, dass** das gemischte Calcium-/NatriumTeilsalz eines Copolymers von Methylvinylether und Maleinsäureanhydrid und die Carboxymethylcellulose in einem Gewichtsverhältnis zwischen 2,8:1 und 1,5:1 vorliegen.

5. Zusammensetzung zur topischen Verwendung nach Anspruch 1, **dadurch gekennzeichnet, dass** Vaseline und Vaselinöl in einem Gewichtsverhältnis zwischen 1,5:1 und 1:1 vorliegen.

6. Verfahren zur Herstellung einer topischen haftenden Zusammensetzung zur therapeutischen Behandlung von Aphthen der Mundschleimhaut, wobei die Zusammensetzung eine wasserfreie Paste ist und das Verfahren aus den folgenden Schritten besteht:
I) Mischen der folgenden Ingredienzen:
a) 5 bis 45 Gewichtsprozent eines gemischten Calcium-/Natrium-Teilsalzes eines Copolymers von Methylvinylether und Maleinsäureanhydrid;
b) 10 bis 40 Gewichtsprozent Vaselinöl;
c) 10 bis 40 Gewichtsprozent weiße Vaseline;
d) 2 bis 25 Gewichtsprozent Carboxymethylcellulose;
f) 0,5 bis 10 Gewichtsprozent D-Panthenol;
g) 0,1 bis 5 Gewichtsprozent Carboxymethylbetaglucan;
h) 0,1 bis 5 Gewichtsprozent gefriergetrocknete Aloe vera;
i) 0,1 bis 5 Gewichtsprozent fettlöslichen Malvenextrakt;
j) 0,05 bis 0,3 Gewichtsprozent Natriumbenzoat;
l) 0,01 bis 2 Gewichtsprozent Vitamin-E-Acetat;
m) 0,01 bis 3 Gewichtsprozent Stearyl-Glycyrrhizat;
n) 0,01 bis 2 Gewichtsprozent Vitamin-A-Palmitat;
o) 0,1 bis 5 Gewichtsprozent Zinkoxid;
p) 0,1 bis 2 Gewichtsprozent Chlorbutanol;
q) 0,000001 bis 0,0001 Gewichtsprozent grüner Farbstoff;
r) 0,05 bis 1 Gewichtsprozent Minzaroma.
II) Mikronisieren von:
e) 1 bis 10 Gewichtsprozent Xylit;
III) Zugabe des mikronisierten Xylits zur Ingredienzenmischung des Schritts I) und Mischen.

## Revendications

1. Composition à usage topique dans le traitement thérapeutique d'une ulcération aphteuse de la muqueuse orale, **caractérisée en ce qu'**elle comprend :
- un mélange substantiellement anhydre contenant un sel partiel mixte calcique/sodique d'un copolymère de méthylvinyléther et d'anhydride maléique ;
- de la carboxyméthylcellulose ;
- de l'huile de vaseline et de la vaseline blanche ;
- du xylitol ;
- du chlorobutanol et du benzoate de sodium ;
- de l'extrait de mauve et du glycyrrhizate de stéaryle ;
- de l'acétate de vitamine E, du palmitate de vitamine A, et du D-panthénol ;
- du carboxyméthylbêtaglucane, de l'aloe vera lyophilisé, de l'oxyde de zinc ;
- des adjuvants comprenant des colorants et des agents aromatisants.

2. Composition à usage topique selon la revendication 1, **caractérisée en ce qu'**elle contient (exprimé en pourcentages en poids) :
a) 5 à 45 % en poids dudit mélange substantiellement anhydre contenant le sel partiel mixte calcique/sodique d'un copolymère de méthylvinyléther et d'anhydride maléique ;
b) 10 à 40 % en poids d'huile de vaseline ;
c) 10 à 40 % en poids de vaseline blanche ;
d) 2 à 25 % en poids de carboxyméthylcellulose ;
e) 1 à 10 % en poids de xylitol ;
f) 0,5 à 10 % en poids de d-panthénol ;
g) 0,1 à 5 % en poids de carboxyméthylbêtaglucane ;
h) 0,1 à 5 % en poids d'aloe vera lyophilisé ;
i) 0,1 à 5 % en poids d'extrait de mauve liposoluble ;
j) 0,05 à 0,3 % en poids de benzoate de sodium ;
l) 0,01 à 2 % en poids d'acétate de vitamine E ;
m) 0,01 à 3 % en poids de glycyrrhizate de stéaryle ;
n) 0,01 à 2 % en poids de palmitate de vitamine A ;
o) 0,1 à 5 % en poids d'oxyde de zinc ;
p) 0,1 à 2 % en poids de chlorobutanol ;
q) 0,000001 à 0,0001 % en poids de colorant vert ;
r) 0,05 à 1 % en poids d'arôme de menthe.

3. Composition à usage topique selon la revendication 2, **caractérisée en ce qu'**elle contient (en formulation qualitative-quantitative pour 100 grammes) :
a) sel partiel mixte calcique/sodique d'un copolymère de méthylvinyléther et d'anhydride maléique = 25 g ;
b) huile de vaseline = 28,65 g ;
c) vaseline blanche = 24 g ;
d) carboxyméthylcellulose = 10 g ;
e) xylitol = 2 g ;
f) d-panthénol = 5 g ;
g) carboxyméthylbêtaglucane = 0,2 g ;
h) aloe vera lyophilisé = 1 g ;
i) extrait de mauve liposoluble = 2 g ;
j) benzoate de sodium = 0,25 g ;
l) acétate de vitamine E = 0,5 g ;
m) glycyrrhizate de stéaryle = 0,3 g ;
n) palmitate de vitamine A = 0,1 g ;
o) oxyde de zinc = 0,2 g ;
p) chlorobutanol = 0,5 g ;
q) colorant vert = 0,0001 g ;
r) arôme de menthe = 0,3 g.

4. Composition à usage topique selon la revendication 1, **caractérisée en ce que** le sel partiel mixte calcique/sodique d'un copolymère de méthylvinyléther et d'anhydride maléique et la carboxyméthylcellulose sont présents en un rapport en poids entre 2,8/1 et 1,5/1.

5. Composition à usage topique selon la revendication 1, **caractérisée en ce que** la vaseline et l'huile de vaseline sont présentes en un rapport en poids entre 1,5/1 et 1/1.

6. Procédé pour la préparation d'une composition adhésive à usage topique dans le traitement thérapeutique d'une ulcération aphteuse de la muqueuse orale sous la forme d'une pâte anhydre, consistant en les étapes suivantes :
I) mélange des ingrédients suivants :
a) 5 à 45 % en poids d'un sel partiel mixte calcique/sodique d'un copolymère de méthylvinyléther et d'anhydride maléique ;
b) 10 à 40 % en poids d'huile de vaseline ;
c) 10 à 40 % en poids de vaseline blanche ;
d) 2 à 25 % en poids de carboxyméthylcellulose ;
f) 0,5 à 10 % en poids de d-panthénol ;
g) 0,1 à 5 % en poids de carboxyméthylbêtaglucane ;
h) 0,1 à 5 % en poids d'aloe vera lyophilisé ;
i) 0,1 à 5 % en poids d'extrait de mauve liposoluble ;
j) 0,05 à 0,3 % en poids de benzoate de sodium ;
l) 0,01 à 2 % en poids d'acétate de vitamine E ;
m) 0,01 à 3 % en poids de glycyrrhizate de stéaryle ;
n) 0,01 à 2 % en poids de palmitate de vitamine A ;
o) 0,1 à 5 % en poids d'oxyde de zinc ;
p) 0,1 à 2 % en poids de chlorobutanol ;
q) 0,000001 à 0,0001 % en poids de colorant vert ;
r) 0,05 à 1 % en poids d'arôme de menthe ;
II) micronisation de
e) 1 à 10 % en poids de xylitol ;
III) addition du xylitol micronisé au mélange d'ingrédients de l'étape I), et mélange.
